(19)

Europäisches Patentamt

European Patent Office

Office européen des brevets

(11) **EP 4 528 573 A1**

(12) **EUROPEAN PATENT APPLICATION**
published in accordance with Art. 153(4) EPC

(43) Date of publication:
26.03.2025 Bulletin 2025/13

(21) Application number: 22859955.1

(22) Date of filing: 20.05.2022

(51) International Patent Classification (IPC):
$G06F\ 30/27^{(2020.01)}$ $G01N\ 1/00^{(2006.01)}$
$G01N\ 33/00^{(2006.01)}$

(52) Cooperative Patent Classification (CPC):
G01N 1/00; G01N 33/00; G06F 30/27

(86) International application number:
PCT/CN2022/094288

(87) International publication number:
WO 2023/024607 (02.03.2023 Gazette 2023/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Favorsun Medical Tech. (Suzhou) Co.,
Ltd.
Suzhou, Jiangsu 215000 (CN)

(72) Inventors:
• LIU, Yezhuo
  Suzhou, Jiangsu 215000 (CN)
• YANG, Wenhua
  Suzhou, Jiangsu 215000 (CN)

(74) Representative: Gong, Jinping
CocreateIP
Neumarkterstr. 21
81673 München (DE)

(54) **METHOD FOR CALCULATING MOLECULAR WEIGHT OF BIO-MACROMOLECULAR MATERIAL ON THE BASIS OF AI ALGORITHM**

(57) The present invention pertains to a method for calculating the molecular weight of biopolymer substances utilizing AI-based computation. Certain biopolymer substances tend not to produce individual biomacromolecular chains when immersed in water-based solutions. This invention employs ionic liquids as the dissolving medium, enabling biomacromolecular substances to form distinct single biomacromolecular chains upon being dissolved. Subsequent to this, rheological techniques are applied to gather rheological data for the biopolymer in ionic liquid solutions. The Rouse model is utilized as a preferred model for characterizing the properties of polymer solution. Through extensive data collection and AI algorithm-assisted nonlinear regression analysis, critical parameter values are derived and employed for calculating the molecular weight of the substance under examination. This approach is characterized by its accuracy and efficiency, suitable for application in polymer material computational research.

Figure 1

EP 4 528 573 A1

## Description

### Field of the Invention

The present invention pertains to an artificial intelligence (AI) method for measuring the molecular weight of a biological macromolecular material, particularly to calculating the molecular weight of materials that are difficult to calculate by a rheological model. This proposed method employs AI-based algorithm to execute a nonlinear fitting procedure for determining the molecular weight of biological materials.

### Background of the Invention

With the continuous development of molecular biology, biological macromolecular materials, such as silk fibroin, hyaluronic acid, collagen, recombinant collagen, and sericin, have received increasing attention. These materials are extensively utilized as crucial components in medical and cosmetic products. The inherent biological characteristics of various biomacromolecular substances are intimately related to their molecular weights, with biomacromolecular substances exhibiting distinct physiological functions depending on their varying molecular weights, and in certain instances, these functions can be entirely dissimilar or even contradictory. For instance, hyaluronic acid possessing a molecular weight exceeding 2000 kDa exhibits good moisturizing, viscoelastic, lubricating, and anti-inflammatory attributes, rendering it appropriate for use in ophthalmic surgeries and joint injections. On the other hand, hyaluronic acid, which has a molecular weight spanning from 10 kDa to 80 kDa, can be ingested and absorbed through the intestines, providing benefits for beauty and improving skin health. Similarly, the increase in molecular weight of silk protein results in a decrease in the swelling and light transmittance of the silk protein hydrogel. Thus, precise assessment of the molecular weight of polymer substances aids in optimizing the utilization of their characteristics.

Conventional approaches for assessing the molecular weight of proteins encompass techniques such as sodium dodecyl sulfate polyacrylamide gel electrophoresis (SDS-PAGE), static light scattering (SLS), small-angle neutron scattering (SANS), gel permeation chromatography (GPC). Nevertheless, these conventional approaches frequently face constraints, particularly with certain polymer materials that are inclined to aggregate when in solution, making precise measurement challenging. For example, silk fibroin molecules, characterized by their distinctive structure, a quasi-multiblock copolymer of hydrophilic and hydrophobic segments, tend to form micelles or aggregate in solution, instead of remaining as individual, unassociated polymer chains. Consequently, identifying an appropriate solvent that allows silk fibroin to remain as discrete single chains is crucial. Then, a fitting analytical technique is employed to quantitatively assess the polymer chains' condition and their viscoelastic properties within the solution. Furthermore, an appropriate polymer solution model is required to resolve these features of the polymeric solution.

To study the structure and dynamic mechanical characteristics of polymer solutions, researchers have established several coarse-grained geometric models, such as the bead-stick model, bead-spring model, pearl necklace model, and reptation model. Further exploration is required to determine which model is most suitable for calculating the molecular weight of silk fibroin.

Determining the molecular weight utilizing those models is inherently intricate, necessitating extensive data and considerable computational power. Hand computations are both slow and prone to errors. The integration of AI algorithms into the conventional approaches for computing polymer molecular weights has led to enhanced punctuality and precision in the calculations, markedly boosting the productivity of researchers and promoting further advancements in biomacromolecular computation research.

### Detailed Description of the Invention

The present invention seeks to overcome the challenges associated with determining the molecular weight of current biopolymer materials, and to develop an AI algorithmic approach for computing the molecular weight of similar biopolymer substances.

The invention discloses a technique for assessing the molecular weight of biopolymers using rheological principles. It employs ionic liquids as the solvent medium and the Rouse model for describing polymer solutions. Through extensive data collection, the AI algorithm refines the model to derive essential parameter values, which are subsequently employed to ascertain the molecular weight of the substance in question.

Firstly, the present invention selects ionic liquids as good solvents for dissolving biomolecules by testing different solvent systems.

Ionic liquids refer to ionic compounds in a liquid state, as well as those with melting points below a specific threshold. In recent years, ionic liquid as a new type of green solvent has attracted the attention of countries all over the world. It possesses several advantageous traits, including high thermal stability, structural tunability, low vapor pressure, non-toxicity, and recyclability, which are beneficial for both environmental protection and the health of operators. Ionic liquids are not only widely used as solvents and reaction media for organic synthesis reactions, but also as green solvents for some natural macromolecules. In terms of dissolving biomolecular materials, ionic liquids also show excellent properties, such as filament protein molecules with unique structures can maintain a single-chain form within ionic liquids.

Secondly, after evaluating numerous polymer solution models via simulation computations, the Rouse model has been identified as particularly fitting for determining the molecular weight of biological polymers. The correlation between the polymer's viscoelasticity data and the molecular weight function within the Rouse model is as indicated below:

$$G' = \sum_i \left[ f(i) \frac{\rho RT}{M_i} \sum_{p=1}^{N} \frac{\omega^2 \tau_{ip}^2}{1 + \omega^2 \tau_{ip}^2} \right] \tag{1}$$

$$G'' = \sum_i \left[ f(i) \frac{\rho RT}{M_i} \sum_{p=1}^{N} \frac{\omega \tau_{ip}}{1 + \omega^2 \tau_{ip}^2} \right] \tag{2}$$

$$\tau_{ip} = \frac{\tau_i^R}{p^2} = \frac{6\eta_0}{\pi^2 p^2 RT} \frac{M_i^2}{M_w} \#(3)$$

Subsequently, the rheological properties of biomacromolecules in ionic liquid solutions were examined using rheological methods. These methods effectively depict the chain condition and viscoelastic properties of polymer chains in solution, enabling the quantification of viscoelastic attributes of polymer chains within ionic liquids.

Finally, this invention employs an AI algorithm to manage the computation of extensive rheological datasets, enhancing both the timeliness and precision of the calculations. This advancement boosts the efficiency of researchers' work and fosters further innovation related to the calculation of the molecular weight of biomacromolecules.

The invention is carried out through the following technical scheme:

The method for calculating molecular weight of biomacromolecular materials based on AI algorithm includes the following steps:

S1. Sample preparation: Dissolve the biopolymer material sample in an ionic liquid to prepare the test solution.

S2. Sample testing: Place the prepared sample from step S1 onto the rheometer to conduct the test and collect the necessary data.

S3. Develop an AI algorithm to evaluate Rouse model.

S4. Formulate the Rouse model to fit the biomacromolecular system.

S5. The Rouse model optimized by AI algorithm was used to calculate the molecular weight of desired biopolymers.

Preferably, in step S1, the specific method for sample preparation is:

a) Preparation of the dissolution system: The dried biopolymer material is combined with the ionic liquid and allowed to dissolve at room temperature;

b) Desiccation and water removal;

c) Decompression and dissolution.

In step S1, it is preferable to use 1-allyl-3-methylimidazole chloride ([AMIm]Cl) and 1-hexyl-3-methylimidazole bisulfate ([HMIm]HSO4).

The tested biomacromolecular substances may encompass silk protein, hyaluronic acid, collagen, recombinant collagen, and sericin. Specifically, the silk protein can be one of mulberry silk protein, spider silk protein, or tussah silk protein. Notably, the silk protein, also referred to as silk fibroin, is derived from the cocoon with sericin removed. For the preparation of the biopolymer solution with ionic liquid, a concentration range of 0.1%-50% was tested, and an optimal concentration of 5-20% was identified for dissolution.

As for the drying and dewatering process in step S1, freeze drying is the chosen method: the sample is subjected to freezing in liquid nitrogen to ensure the complete curing of the ionic liquid, followed by dehydration in the freeze dryer. The fully dried sample is then collected and sealed at room temperature.

Preferably, in the step S1, the detailed process for decompression dissolution involves: heating the silk protein ionic liquid blend crafted in step b under stirring, applying oil bath and vacuum via the oil pump to evaporate any residual moisture in the mixture and to remove bubbles, and continuing to heat the biopolymer until it is entirely dissolved.

Preferably, the blend of ionic liquids with silk fibroin, produced during step S1 (b), was warmed in an oil bath while maintaining a temperature range of 0-180 ° C with continuous stirring. Subsequently, it underwent distillation using an oil pump at a reduced pressure, varying the vacuum level between -0.01MPa and -0.5MPa, to eradicate any remaining trace water within the mixture and to remove air bubbles. This process continued until the biopolymer was fully dissolved. Once this was achieved, heating and stirring were ceased, and the solution was allowed to cool, resulting in a consistent and clear solution.

Preferably, the obtained biopolymer ionic liquid mixture is sealed and stored at room temperature in a dry environment until use.

In step S2 , the specific method for testing the sample with a rheometer is as follows:

a) Energy storage modulus and loss modulus test

Parallel splints are chosen and the test is protected by nitrogen purge of the temperature control hood.

The linear dynamic elasticity examination is applied, maintaining the strain amplitude of the oscillating mode below 50% to guarantee that both the storage modulus and loss modulus remain linear across the frequency sweep span (ranging $1\times10^2$ rad/s to $30\times10^{-2}$ rad/s).

The frequency sweep was conducted at specified temperatures, including 0 °C, 10 °C, 20 °C, and 30 °C, in order to obtain the curves of the energy storage modulus and loss modulus of the sample at different temperatures.

b) Viscosity test

Parallel splints are chosen, and the test is protected by nitrogen purge of the temperature control hood.

The steady-state test experiment was conducted: the shear rate was systematically varied from low to high, spanning a range of $10^{-5}$ to $10^5$ $s^{-1}$. The viscosity values at the plateau curve were meticulously recorded.

In step S3, the method for establishing the AI algorithm to assess Rouse model is preferably as follows: Employing a conventional AI model optimization algorithm, an evaluation algorithm for the Rouse model's predictive quality is created. This algorithm quantifies the concordance between the Rouse model predictions and experimental outcomes. For the fitting, it is imperative that the Rouse model's theoretical predictions closely mirror the experimental results. Therefore, the following algorithm parameters need to be set in advance:

a) Set a reasonable error threshold $\varepsilon_{th}$. When the prediction error $\varepsilon$ of Rouse model is less than $\varepsilon_{th}$, it is considered that the accuracy of Rouse model is high enough to truly describe the experimental polymer system;

b) Set a reasonable initial learning rate $\alpha_0$ to control the iteration speed of the algorithm;

c) The gradient descent method is used to continuously modify the model parameters until the prediction error is less than 0.01.

Preferably, in the step S3, the AI model optimization algorithms include Gradient Descent, Conjugate Descent, Adam, AdamGrad, and RMSProp.

Preferably, in step S3, the error threshold $\varepsilon_{th}$ is selected from 0.001 to 0.25.

Preferably, in step S3, the learning rate $\alpha_0$ is selected in the range 0.0001-0.1.

Preferably, in step S4, the specific method for constructing the Rouse model to fit the biopolymer system is as follows:

a) It is assumed that the structure of the biopolymer system meets the Rouse model, and the molecular weight distribution of the system meets the normal distribution. The experimental system was modeled and the mean $\overline{M}_0$ and standard deviation $\Delta M_0$ of the molecular weight distribution were initialized.

b) Using $\overline{M}_0$ and $\Delta M_0$ to construct the normal distribution $M\sim N(\overline{M}, \Delta M)$ of molecular weight [Formula (4)], and sampling according to this distribution to establish the initial simulated physical system.

$$f(M_i) = \frac{1}{\Delta M \sqrt{2\pi}} e^{-\frac{(M_i - \bar{M})}{2\sigma^2}} \qquad (4)$$

c) According to the molecular weight distribution of the simulated physical system, the relaxation time $\tau_{ip}$ under mode p is calculated according to equation (3).

d) Considering the contribution of all vibration modes p to the storage modulus **G'** and the dissipative modulus **G"**, the **G'** and **G"** of the simulated physical system are calculated according to equations (1) and (2). The density $\rho$ of the polymer in the solution, the zero-cut viscosity $\eta_0$ of the solution and the viscosity $\eta_s$ of the solvent used in the calculation are consistent with the experimental data.

e) According to the simulation data obtained from the Rouse theoretical model, the **log G' ~ log $\omega$** and **log G" ~ log $\omega$** relationships of the simulated physical system can be calculated. The error between the predicted value **log G' and log G"** of the Rouse model and the experimental value measured in step S2 $\varepsilon$ can be solved by the root mean square formula.

f) The error $\varepsilon$ between the calculated **log G' and log G",** which describes how well the Rouse model agrees with the experimental results. The fitting process requires that the prediction results of the Rouse model tend to be close to the experimental measurements. Therefore, the error $\varepsilon$ must be as small as possible, and the fitting of the Rouse model evolves into an optimization problem with the following objectives:

$$\min_{\bar{M}, \Delta M} \varepsilon\big(N(\bar{M}, \Delta M)\big) \qquad (5)$$

The error $\varepsilon$ changes with the size distribution of the polymers, and the objective function of equation (5) is optimized by using the AI algorithm described in step S3. Compare the error threshold of the actual $\varepsilon$ and the $\varepsilon_{th}$ derived from AI algorithm calculation. For example, if it is $\varepsilon \geq \varepsilon_{th}$, the normal distribution parameters $\bar{M}$ and $\Delta M_0$ are optimized and updated by the AI algorithm, and the Rouse model is recalculated. If $\varepsilon < \varepsilon_{th}$ is used, the Rouse model can accurately describe the experimental results. It will complete this step and move on to the next step.

Preferably, in the step S5, the molecular weight is calculated using the Rouse model optimized by an AI algorithm, and the molecular weight-related data are the weight-averaged molecular weight, the number-averaged molecular weight and the molecular weight distribution.

Further, in the step S5, the optimized Rouse model obtained in step S4 is used to complete the calculation of the weight-average molecular weight $M_w$, the number-average molecular weight $M_n$ and the molecular weight distribution $(M_w/M_n)$, by using the molecular weight distribution of the simulated physical system $N(\bar{M}_{opt}, \Delta M_{opt})$.

**Figure legends:**

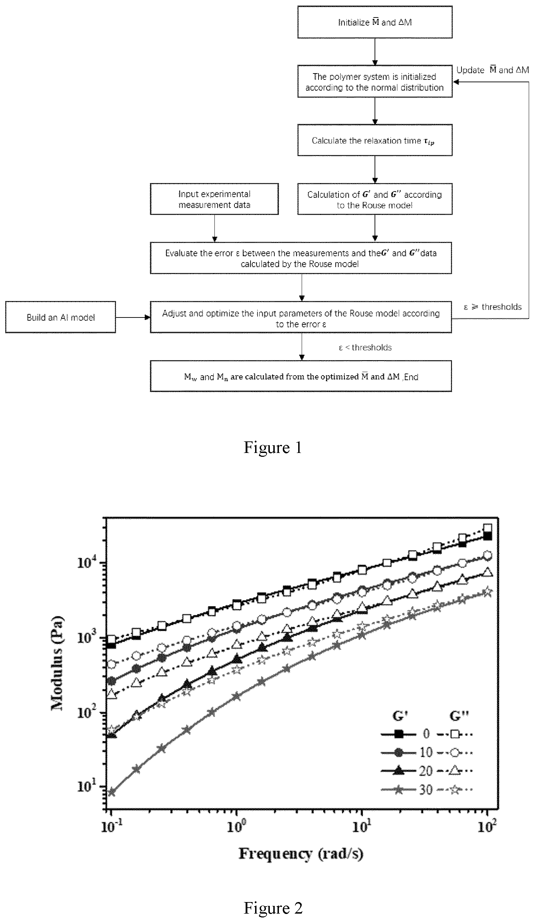

Figure 1. The flow chart of calculating the molecular weight of a by Rouse model.

Figure 2. The correlation between the storage modulus and the loss modulus, along with the shear frequency relative to silk proteins of varying molecular weights, is observed in example 1 of this invention when examining the silk protein ionic liquid solution in oscillatory mode.

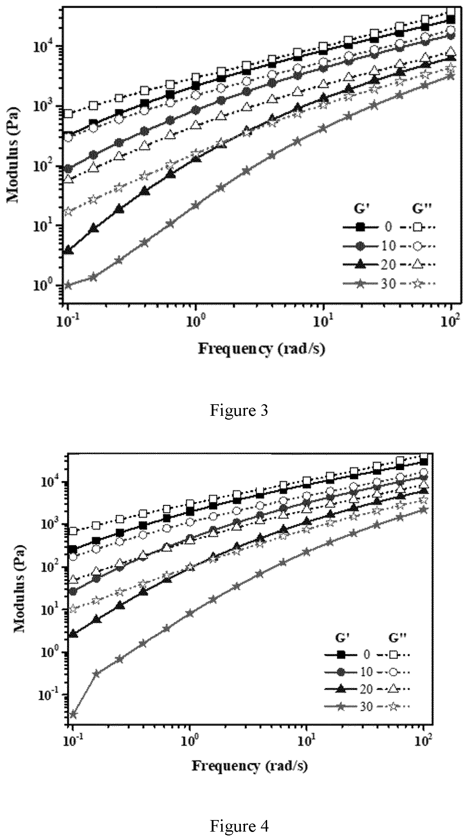

Figure 3 The correlation between the storage modulus and the loss modulus, along with the shear frequency relative to silk proteins of varying molecular weights, is observed in example 2 of this invention when examining the silk protein ionic liquid solution in oscillatory mode.

Figure 4 The correlation between the storage modulus and the loss modulus, along with the shear frequency relative to silk proteins of varying molecular weights, is observed in example 3 of this invention when examining the silk protein ionic liquid solution in oscillatory mode.

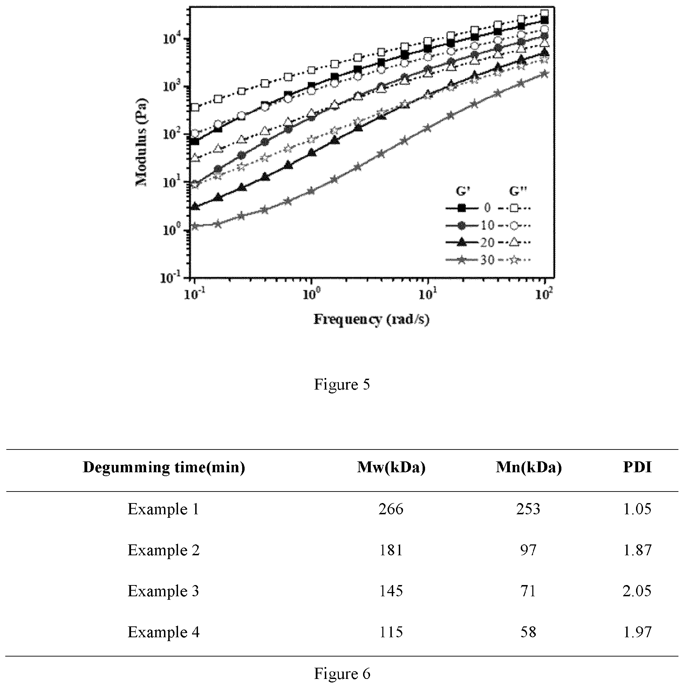

Figure 5 The correlation between the storage modulus and the loss modulus, along with the shear frequency relative to silk proteins of varying molecular weights, is observed in example 4 of this invention when examining the silk protein ionic liquid solution in oscillatory mode.

Figure 6. The analyzed results of the molecular weights of different silk proteins in examples 1- 4.

**The specified examples:**

The present invention is embodied with the following specified examples. However, it is understood by those versed in the field that these detailed examples do not define the boundaries of the present invention's protection.

**Example 1:**

S1: Sample preparation

a) Dissolution: Silkworm silk fibroin prepared by sodium carbonate degumming for 15 minutes was selected, and mulberry silk protein was mixed with ionic liquid 1-allyl-3-methylimidazole chloride salt, and the concentration of silk fibroin was controlled at 20%.

b) Freeze-drying and dehydration: The sample is frozen in liquid nitrogen to fully solidify the ionic liquid, and placed in a freeze-dryer to be fully dehumidified and then taken out, sealed and thawed at room temperature.

c) Dissolution under reduced pressure: The silk fibroin ionic liquid mixture prepared by step b is heated in an oil bath at a temperature of 150 °C under stirring, and distilled with an oil pump under reduced pressure (vacuum range: -0.01 MPa ~ -0.5 MPa) to remove the possible residual trace water in the mixture and eliminate air bubbles, and heat until the silk fibroin is completely dissolved. Stop heating and stirring, and get a uniform and transparent solution after cooling. The resulting silk fibroin ionic liquid solution is stored at room temperature in a sealed and dry environment for later use.

S2: Rheological Tests:

1) Energy storage modulus and loss modulus test

Parallel splints are chosen, and the test is protected by nitrogen purge of the temperature control hood.
The linear dynamic elasticity test is adopted, that is, the strain amplitude of the oscillation mode is controlled below 50% to ensure that the storage modulus and loss modulus are linear in the frequency sweep range ($1\times10^2$ rad/s ~$30\times10^{-2}$ rad/s).
Frequency sweeps were performed at the following temperatures (0 °C, 10 °C, 20 °C, and 30 °C) to obtain the storage modulus and loss modulus curves of the samples at different temperatures.

2) Viscosity test

Parallel splints are chosen, and the test is protected by nitrogen purge of the temperature control hood.
Steady-state test experiments were adopted: the shear rate was scanned from low to high, and the shear rate ranged from $10^{-5}$ ~$10^5$ s$^{-1}$. The viscosity value of the platform curve is recorded.
S3: Modeling with the Rouse Model: Based on the commonly used AI model optimization algorithms, an algorithm is established to evaluate the quality of the prediction results of the Rouse model.

a) Set the error threshold $\varepsilon_{th}$=0.01, when the prediction error of the Rouse model is $\varepsilon$ less than $\varepsilon_{th}$ it is considered that the accuracy of the Rouse model is high enough to truly describe the experimental polymer system.

b) Set the initial learning rate $\alpha0$=0.05 to control the iteration speed of the algorithm.

C) The gradient descent method was used to continuously modify the model parameters until the prediction error was less than 0.01.

S4: The specific method of establishing the Rouse model to fit the biopolymer system is as follows: a) It is assumed that the structure of the biopolymer system satisfies the Rouse model, and the molecular weight distribution of the system satisfies the normal distribution. The experimental system was modeled, and the mean $\overline{M}_0$ and standard deviation $\Delta M_0$ of the molecular weight distribution were initialized.

b) $\overline{M}_0$ and $\Delta M_0$ were used to construct a normal distribution of molecular weight $M\sim N(\overline{M}, \Delta M)$ [Equation (4)], and the initial simulation physics system was established by sampling according to this distribution

$$f(M_i) = \frac{1}{\Delta M\sqrt{2\pi}}e^{-\frac{(M_i-\overline{M})}{2\sigma^2}} \qquad (4)$$

c) According to the molecular weight distribution of the simulated physical system, the relaxation time $\tau_{ip}$ under mode p is calculated according to equation (3).

d) Considering the contribution of all vibration modes p to the storage modulus G' and the dissipative modulus G", the G' and G" of the simulated physical system are calculated according to equations (1) and (2). The density $\rho$ of the polymer in the solution, the zero-cut viscosity $\eta 0$ of the solution and the viscosity $\eta s$ of the solvent used in the calculation are consistent with the experimental data.

e) According to the simulation data obtained from the Rouse theoretical model, the relationship between *log G'~ log ω* and *log G"~ log ω* of the simulated physical system can be calculated. The error ε between the predicted values **log G'** and **log G"** of the Rouse model and the experimental value measured in step S2 can be solved by the root mean square formula.

f) The calculated error $\varepsilon$ of the **log G' and log G",** which describes the degree to which the Rouse model agrees with the experimental results. The fitting process requires that the prediction results of the Rouse model tend to be close to the experimental measurements. Therefore, the error $\varepsilon$ must be as small as possible, and the fitting of the Rouse model evolves into an optimization problem with the optimization objectives as:

$$\min_{\overline{M},\Delta M} \varepsilon\big(N(\overline{M},\Delta M)\big) \qquad (5)$$

The error $\varepsilon$ varies with the distribution of the polymer, and the objective function of equation (5) is optimized by using the AI algorithm described in step S3. Compare the error threshold $\varepsilon_{th}$ by the AI algorithm of the actual $\varepsilon$, for example, $\varepsilon \geq \varepsilon_{th}$, the normal distribution parameters $\overline{M}$ and $\Delta M$ are optimized and updated by the AI algorithm, and the process b is returned after the update), and the Rouse model is recalculated. For example, $\varepsilon < \varepsilon_{th}$ means that the Rouse model at this time has been able to accurately describe the experimental results, complete this step and move on to the next step.

S5: Calculate molecular weight-related data using the Rouse model optimized by AI algorithm: The optimized Rouse model obtained in step S4 is used to simulate the molecular weight distribution $N(\overline{M}_{opt}, \Delta M_{opt})$. of the physical system to achieve a weight-average molecular weight $M_w$ of 266 kDa, a number-average molecular weight $M_n$ of 253 kDa, and a molecular weight distribution of 1.05.

Example 2

S1: Sample Preparation:

a) Dissolution: Silkworm silk fibroin prepared by sodium carbonate degumming for 30 minutes was selected, and Silkworm silk fibroin was mixed with ionic liquid 1-allylenyl-3-methylimidazole chloride salt, and the concentration of silk fibroin was controlled at 20%.

b) Freeze-drying and dehydration: The sample is frozen in liquid nitrogen to fully solidify the ionic liquid, and placed in a freeze-dryer to be fully dehumidified and then taken out, sealed and thawed at room temperature.

c) Dissolution under reduced pressure: The silk fibroin ionic liquid mixture prepared by step b is heated in an oil bath at a temperature of 150 °C under stirring and distilled under reduced pressure with an oil pump (vacuum range: -0.01 MPa ~ -0.5 MPa) to remove the possible residual trace water in the mixture and eliminate air bubbles, and heat until the silk fibroin is completely dissolved. Stop heating and stirring, and get a uniform and transparent solution after cooling. The resulting silk fibroin ionic liquid solution is stored at room temperature in a sealed and dry environment for later use.

S2: Rheological method test: Same as step S2 in example 1.
S3: modeling with a Rouse model: Same as step S3 in example 1.
S4: Establish a Rouse model to fit the biopolymer system: Same as step S4 in example 1.

S5: Calculate molecular weight-related data using the Rouse model optimized by AI algorithm: The optimized Ruse model obtained in step S4 is used to simulate the molecular weight distribution $N(\overline{M}_{opt}, \Delta M_{opt})$ of the physical system to achieve a weight-average molecular weight $M_w$ of 181 kDa, a number-average molecular weight $M_n$ of 97 kDa, and a molecular weight distribution of 1.87.

Example 3

S1: Sample Preparation:

a) Dissolution: The soluble large molecular weight spray-dried silkworm silk fibroin powder prepared by degumming with sodium carbonate for 45 minutes is used. The silkworm silk fibroin powder is mixed with ionic liquid 1-allyl-3-methylimidazolium chloride, and the concentration of silk fibroin is controlled to 20%.

b) Freeze-drying and dehydration: The sample is frozen in liquid nitrogen to fully solidify the ionic liquid, and placed in a freeze-dryer to be fully dehumidified and then taken out, sealed and thawed at room temperature.

c) Dissolution under reduced pressure: The silk fibroin ionic liquid mixture prepared by step b is heated in an oil bath at a temperature of 120 °C under stirring, and distilled under reduced pressure with an oil pump (vacuum range: -0.01 MPa ~ -0.5 MPa) to remove the possible residual trace water in the mixture and eliminate air bubbles, and heat until the silk fibroin is completely dissolved. Stop heating and stirring, and get a uniform and transparent solution after cooling. The resulting silk fibroin ionic liquid solution is stored at room temperature in a sealed and dry environment for later use.

S2: Rheological method test: Same as step S2 in example 1.
S3: modeling with a rouse model: Same as step S3 in example 1.
S4: Establish a Rouse model to fit the biopolymer system: Same as step S4 in example 1.
S5: Calculate molecular weight-related data using the Rouse model optimized by AI algorithm: The optimized Rouse model obtained in step S4 is used to simulate the molecular weight distribution $N(\overline{M}_{opt}, \Delta M_{opt})$. of the physical system to achieve a weight-average molecular weight $M_w$ of 145 kDa, a number-average molecular weight $M_n$ of 71 kDa, and a molecular weight distribution of 2.05.

Example 4

S1: Sample Preparation:

a) Dissolution: The soluble large molecular weight freeze-dried silkworm silk fibroin powder prepared by sodium carbonate degumming for 60 minutes was selected, and the silkworm silk fibroin powder was mixed with ionic liquid 1-allyl-3-methylimidazole chloride salt, and the concentration of silk fibroin was controlled at 20%.

b) Freeze-drying and dehydration: The sample is frozen in liquid nitrogen to fully solidify the ionic liquid, and placed in a freeze-dryer to be fully dehumidified and then taken out, sealed and thawed at room temperature.

c) Dissolution under reduced pressure: The silk fibroin ionic liquid mixture prepared by step b is heated in an oil bath at a temperature of 120 °C under stirring, and distilled under reduced pressure with an oil pump (vacuum range: -0.01 MPa ~ -0.5 MPa) to remove the possible residual trace water in the mixture and eliminate air bubbles, and heat until the silk fibroin is completely dissolved. Stop heating and stirring, and get a uniform and transparent solution after cooling. The resulting silk fibroin ionic liquid solution is stored at room temperature in a sealed and dry environment for later use.

S2: Rheological method test: Same as step S2 in example 1.
S3: modeling with a rouse model: Same as step S3 in example 1.
S4: Establish a Rouse model to fit the biopolymer system: Same as step S4 in example 1.
S5: Molecular weight-related data are calculated using the Rouse model optimized by AI algorithm: The optimized Rouse model obtained in step S4 is used to simulate the molecular weight distribution $N(\overline{M}_{opt}, \Delta M_{opt})$. of

the physical system to achieve a weight-average molecular weight $M_w$ of 115 kDa, a number-average molecular weight $M_n$ of 58 kDa, and a molecular weight distribution of 1.97.

**Claims**

1. A method for calculating the molecular weight of biopolymer materials based on AI algorithms and the following steps:

   S1: Sample Preparation: Take the biopolymer material sample to be tested, dissolve it in an ionic liquid to obtain the sample solution, which is the ionic liquid solution of the biopolymer material.
   S2: Sample Testing: Place the sample prepared in step S1 on a rheometer for sample testing and calculate the required data.
   S3: Establish AI Algorithm to Evaluate Rouse Model: Using common AI model optimization algorithms, establish an algorithm to assess the quality of the Rouse model's prediction results.
   S4: Fit the Rouse Model to the Biopolymer solution system.
   S5: Use the AI-optimized Rouse model to calculate the molecular weight of biopolymers.

2. The method for calculating the molecular weight of a biological macromolecule material based on an AI algorithm according to claim 1, in which the particular technique for sample preparation in step S1 is as follows:

   a) Preparation of the dissolution system: The dried biopolymer material sample is dissolved into the ionic liquid and dissolved at room temperature;
   b) Desiccation and water removal;
   c) Decompression and dissolution, in step S1, the biopolymer material includes silk fibroin, hyaluronic acid, collagen, recombinant collagen, and fibroin protein, the silk fibroin can be selected from options like silkworm silk, spider silk, or tussah silk, the ionic liquid used in step S1 is the combination of [AMIm]Cl and [HMIm]HSO4.

3. The method for calculating the molecular weight of the biomacromolecular material based on the AI algorithm according to claim 1 or 2, wherein the concentration of the silk fibroin in the ionic liquid solution of the silk protein of the biopolymer material prepared in the step S1 is 0.1%-50%, 1%-20%, or 5-15%.

4. The method for calculating the molecular weight of a biological macromolecule material based on an AI algorithm as claimed in claim 2, wherein the freeze-drying method is selected in the step S1 to dry and remove water. Specifically, the sample is placed in liquid nitrogen for freezing and is placed in a freeze-dryer to prepare the lyophilized powder. It was then taken out after being fully dehumidified and sealed for storage at room temperature.

5. The approach for determining the molecular weight of biopolymer materials using AI algorithms as described in claim 2 or 4 is defined by the particular vacuum dissolution technique: the blend of silk fibroin and ionic liquid made in step S1(b) is warmed while being stirred in an oil bath, then undergoes vacuum distillation with an oil pump to get rid of any remaining water and to eradicate bubbles, continuing the heating process until the biopolymer is fully dissolved, the temperature of oil bath is 0-180°C, and the vacuum range is -0.01 MPa to -0.5 MPa, to remove any residual water and eliminate bubbles. The heating process persists as the biopolymer is fully dissolved. Upon ceasing the heating and mixing process, the solution is permitted to cool, subsequently transforming into a consistent and clear liquid, the obtained biopolymer ionic liquid solution is sealed, and stored in a dry environment at room temperature for later application.

6. The technique for determining the molecular weight of biopolymer substances using AI algorithms as per claims 1-5 is distinguished by the particular approach for sample examination in step S2:

   a) Storage Modulus and Loss Modulus Testing: Parallel plates are chosen, and safeguard the testing environment by introducing nitrogen gas through a temperature-regulated cover during the process; employ linear dynamic elasticity measurements where the strain amplitude is maintained below 50%, guaranteeing the linearity of the storage and loss moduli across the frequency sweep spectrum (from $1 \times 10^2$ rad/s - $30 \times 10^{-2}$ rad/s), conduct frequency sweeps at varying temperatures (0°C, 10°C, 20°C, and 30°C) to generate storage and loss modulus curves corresponding to different temperatures;
   b) Viscosity Assessment: Choose parallel plates and shield the test from the nitrogen gas stream at a steady temperature; perform steady-state tests by scanning the shear rate from low to high, within the range of $10^{-5}$ - $10^5$ $s^{-1}$, and record the viscosity values by the instrument.

7. The method for calculating the molecular weight of biopolymer materials utilizing AI algorithms as per claims 1-6 is distinguished by the particular approach for constructing the AI algorithm to assess the Rouse model during step S3, which is outlined as follows:

a) Set a suitable error threshold $\varepsilon_{th}$, where the Rouse model's prediction error $\varepsilon$ is considered acceptable if below the threshold $\varepsilon_{th}$, suggesting that the Rouse model is adequately precise for characterizing the experimental polymer system;
b) Set a reasonable initial learning rate $\alpha_0$ to control the iteration speed of the algorithm;
c) Employ gradient descent to continuously adjust the model parameters until the prediction error is below 0.01, the optimization algorithms of AI model in step S3 include Gradient Descent, Conjugate Descent, Adam, AdamGrad, and RMSProp.

8. The method for calculating the molecular weight of biopolymer materials based on AI algorithms according to claims 1-7 is distinguished by selecting the error threshold $\varepsilon_{th}$ in step S3 within the bracket of 0.001-0.25, and by opting for the learning rate $\alpha_0$ within the confines of 0.0001-0.1.

9. The method for calculating the molecular weight of biopolymer materials based on AI algorithms according to claims 1-8, **characterized in that** the specific method for fitting the Rouse model to the biopolymer system in step S4 is as follows:

a) It is assumed that the structure of the biomolecular system meets the Rouse model, and the molecular weight distribution of the system meets the normal distribution; The experimental system was modeled, and the mean $\overline{M}_0$ and standard deviation $\Delta M_0$ of molecular weight distribution were initialized;
b) $\overline{M}_0$ and $\Delta M_0$ were used to construct a normal distribution of molecular weight $M \sim N(\overline{M}, \Delta M)$ [Formula (4)], and samples were taken according to this distribution to establish an initial simulated physical system;

$$f(M_i) = \frac{1}{\Delta M \sqrt{2\pi}} e^{-\frac{(M_i - \overline{M})}{2\sigma^2}} \qquad (4)$$

c) Calculate the relaxation time $\tau_{ip}$ of mode p based on the molecular weight distribution of the simulation physical system according to formula (3);
d) Consider the contributions of all vibration modes p to the storage modulus $G'$ and dissipation modulus $G''$ and calculate $G'$ and $G''$ of the simulated physical system according to formulas (1) and (2),the polymer density $\rho$ in solution, the zero shear viscosity $\eta_o$, and the solvent viscosity $\eta_s$ used in the calculation should be consistent with experimental data;
e) According to the simulated data obtained from the Rouse theoretical model, the relationship between $\log G' \sim \log \omega$ and $\log G'' \sim \log \omega$ of the simulated physical system can be calculated. The error $\varepsilon$ between the predicted values $\log G'$ and $\log G''$ of the Rouse model and the experimental values measured in step S2 can be solved by the root-mean-square formula.
f) The error $\varepsilon$ between the calculated $\log G'$ and $\log G''$, which describes the degree of agreement between the Rouse model and the experimental results, the fitting process requires that the prediction results of Rouse model approximate to the experimental measurement results. Therefore, the error $\varepsilon$ must be as small as possible, then the fitting of the Rouse model is an optimization problem, and the optimization objective is as follows:

$$\min_{\overline{M}, \Delta M} \varepsilon\big(N(\overline{M}, \Delta M)\big) \qquad (5)$$

The error $\varepsilon$ varies with the distribution of polymers. The AI algorithm described in Step S3 is used to optimize the objective function of formula (5). Compare the error threshold error $\varepsilon$ of the error $\varepsilon$ and AI algorithm, such as $\varepsilon \geq \varepsilon_{th}$, then optimize and update the normal distribution parameters $\overline{M}$ and $\Delta M$ by AI algorithm, and return to process b) after updating, and re-calculate the Rouse model; If $\varepsilon < \varepsilon_{th}$, it indicates that the Rouse model can accurately describe the experimental results.

10. The method for calculating the molecular weight of biopolymer materials based on AI algorithms according to claim 1, **characterized in that** in step S5, the molecular weight data includes weight-average molecular weight, number-average molecular weight, and molecular weight distribution, in step S5, the method for calculating the molecular weight using the AI-optimized Rouse model is: using the optimized Rouse model obtained from steps S1-S4, the molecular weight distribution $N(\overline{M}_{opt}, \Delta M_{opt})$. of the simulation physical system is used to calculate the weight-average molecular weight $M_w$, number-average molecular weight $M_n$, and molecular weight distribution $M_w/M_n$.

Figure 1

Figure 2

Figure 3

Figure 4

Figure 5

| Degumming time(min) | Mw(kDa) | Mn(kDa) | PDI |
|---|---|---|---|
| Example 1 | 266 | 253 | 1.05 |
| Example 2 | 181 | 97 | 1.87 |
| Example 3 | 145 | 71 | 2.05 |
| Example 4 | 115 | 58 | 1.97 |

Figure 6

## INTERNATIONAL SEARCH REPORT

International application No.

**PCT/CN2022/094288**

### A. CLASSIFICATION OF SUBJECT MATTER

G06F 30/27(2020.01)i; G01N 1/00(2006.01)i; G01N 33/00(2006.01)i

According to International Patent Classification (IPC) or to both national classification and IPC

### B. FIELDS SEARCHED

Minimum documentation searched (classification system followed by classification symbols)

G06F; G01N

Documentation searched other than minimum documentation to the extent that such documents are included in the fields searched

Electronic data base consulted during the international search (name of data base and, where practicable, search terms used)

CNPAT, CNKI, WPI, EPODOC: 人工智能, AI, 大分子, 高分子, 分子量, 样品, 溶液, 流变仪, Rouse, 模型, 误差, 阈值, Artificial Intelligence, macromolecule, molecular weight, sample, solution, rheometer, model, error, threshold

### C. DOCUMENTS CONSIDERED TO BE RELEVANT

| Category* | Citation of document, with indication, where appropriate, of the relevant passages | Relevant to claim No. |
|---|---|---|
| A | CN 103234868 A (CHANGZHOU UNIVERSITY) 07 August 2013 (2013-08-07) description, paragraphs 0003-0015 | 1-10 |
| A | JP 2016118501 A (SUMITOMO RUBBER INDUSTRY LTD.) 30 June 2016 (2016-06-30) entire document | 1-10 |
| A | 杜丽君等 (DU, Lijun et al.). "流变法在氟塑料分子量及其分布测试中的应用 (Non-official translation: Application of Rheological Method in Molecular Weight and Distribution Test of Fluoroplastics)" 有机氟工业 (Organo-Fluorine Industry), No. 01, 15 March 2014 (2014-03-15), entire document | 1-10 |
| A | 江体乾 (JIANG, Tiqian). "多分散聚合物稀溶液体系的Rouse模型 (Non-official translation: Rouse Model of Polydisperse Polymer Dilute Solution System)" 化工学报 (CIESC Journal), No. 04, 25 August 1989 (1989-08-25), entire document | 1-10 |
| A | US 2015212101 A1 (LABORATORIOS SANIFIT, S.L.) 30 July 2015 (2015-07-30) entire document | 1-10 |

☐ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **16 November 2022** | **30 November 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

| International application No. |
| --- |
| **PCT/CN2022/094288** |

| Patent document cited in search report | | | Publication date (day/month/year) | Patent family member(s) | | | Publication date (day/month/year) |
| --- | --- | --- | --- | --- | --- | --- | --- |
| CN | 103234868 | A | 07 August 2013 | None | | | |
| JP | 2016118501 | A | 30 June 2016 | None | | | |
| US | 2015212101 | A1 | 30 July 2015 | DK | 2764360 | T3 | 25 March 2019 |
| | | | | EP | 2579036 | A1 | 10 April 2013 |
| | | | | US | 2017254823 | A1 | 07 September 2017 |
| | | | | EP | 3499232 | A1 | 19 June 2019 |
| | | | | PT | 2764360 | T | 18 March 2019 |
| | | | | JP | 2014533353 | A | 11 December 2014 |
| | | | | WO | 2013050603 | A1 | 11 April 2013 |
| | | | | US | 2022187326 | A1 | 16 June 2022 |
| | | | | ES | 2714402 | T3 | 28 May 2019 |
| | | | | EP | 2764360 | A1 | 13 August 2014 |

Form PCT/ISA/210 (patent family annex) (January 2015)